(19) Europäisches Patentamt
European Patent Office
Office européen des brevets

(11) Veröffentlichungsnummer: **0 296 264**
**A1**

(12) # EUROPÄISCHE PATENTANMELDUNG

(21) Anmeldenummer: **87109015.5**

(22) Anmeldetag: **23.06.87**

(51) Int. Cl.⁴: **C07C 93/06 , C07D 295/08 , A61K 31/13 , A61K 31/395**

(43) Veröffentlichungstag der Anmeldung:
**28.12.88 Patentblatt 88/52**

(84) Benannte Vertragsstaaten:
**AT BE CH DE ES FR GB GR IT LI LU NL SE**

(71) Anmelder: **Helopharm W. Petrik GmbH & Co.KG.**
**Waldstrasse 23-25**
**D-1000 Berlin 51(DE)**

(72) Erfinder: **Petrik, Gerd**
**Herthastrasse 11**
**D.1000 Berlin 33(DE)**
Erfinder: **Klemens, Schubert, Dr.**
**Eichborndamm 5**
**D-1000 Berlin 51(DE)**

(74) Vertreter: **Vossius & Partner**
**Siebertstrasse 4 P.O. Box 86 07 67**
**D-8000 München 86(DE)**

(54) **Aminopropanolderivate von 3-(2-Hydroxyphenyl)-1-propanonderivaten, Verfahren zu ihrer Herstellung und diese Verbindungen enthaltende Arzneimittel.**

(57) Beschrieben sind Aminopropanolderivate von 3-(2-Hydroxyphenyl)-1-propanonderivaten der allgemeinen Formel I

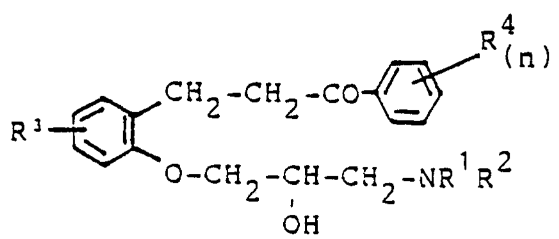

(I)

und ihre Säureadditionssalze, ein Verfahren zu ihrer Herstellung und diese Verbindungen enthaltende Arzneimittel.

EP 0 296 264 A1

**" Aminopropanolderivate von 3-(2-Hydroxyphenyl)-1-propanonderivaten, Verfahren zu ihrer Herstellung und diese Verbindungen enthaltende Arzneimittel "**

Die Erfindung betrifft neue Aminopropanolderivate von 3-(2-Hydroxyphenyl)-1-propanonderivaten der allgemeinen Formel I

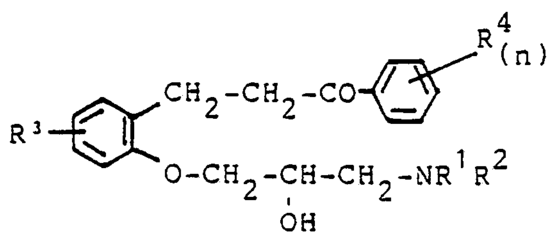

(I)

und ihre Säureadditionssalze sowie ein Verfahren zu ihrer Herstellung. Die Erfindung betrifft ferner Arzneimittel, die eine Verbindung der allgemeinen Formel I oder deren Säureadditionssalz, vorzugsweise ein physiologisch verträgliches Säureadditionssalz, enthalten.

In der allgemeinen Formel I sind $R^1$ und $R^2$ gleich oder verschieden und bedeuten Wasserstoffatome, Alkyl-, Cycloalkyl-, Alkenyl-, Alkinyl- oder Hydroxyalkylreste mit jeweils bis zu 6 C-Atomen, Alkoxyalkyl-, Alkylthioalkyl- oder Dialkylaminoalkylreste mit jeweils bis zu 9 C-Atomen, oder Phenylalkyl- oder Phenoxyalkylreste mit bis zu 6 C-Atomen im Alkylteil, wobei gegebenenfalls der Phenylrest durch einen Alkyl- oder Alkoxyrest mit jeweils bis zu 3 C-Atomen substituiert ist, oder

$R^1$ und $R^2$ bilden zusammen mit dem sie verbindenden Stickstoffatom einen 5- bis 7-gliedrigen gesättigten heterocyclischen Ring, der gegebenenfalls durch einen oder zwei Phenyl- und/oder Hydroxylreste substituiert sein und ein Sauerstoffatom als weiteres Heteroatom im Ring enthalten kann, wobei ein zusätzliches Stickstoffatom durch einen Alkylrest mit 1 bis 3 C-Atomen oder einen Phenylrest substituiert sein kann, $R^3$ ist ein Wasserstoffatom, ein Alkylrest mit bis zu 3 C-Atomen, ein Fluor-, Chlor- oder Bromatom, eine Hydroxylgruppe oder eine Alkoxygruppe mit bis zu 6 C-Atomen. $R^4$ ist ein Wasserstoffatom, ein Alkylrest mit bis zu 3 C-Atomen, ein Fluor-, Chlor- oder Bromatom, eine Hydroxylgruppe oder eine Alkoxygruppe mit bis zu 6 C-Atomen. n ist eine ganze Zahl mit einem Wert von 1 bis 5.

Die Erfindung betrifft ferner ein Verfahren zur Herstellung der Verbindungen der allgemeinen Formel I und ihrer Säureadditionssalze, das dadurch gekennzeichnet ist, daß man einen Phenoläther der allgemeinen Formel II

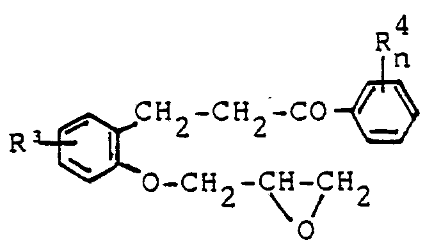

(II)

mit einem Amin der allgemeinen Formel III

$HNR^1R^2$ (III)

umsetzt.

Gegebenenfalls wird die erhaltene Verbindung der allgemeinen Formel I mit einer Säure in ein Säureadditionssalz überführt. Die Umsetzung kann beispielsweise nach dem in der EP-PS 0 074 014 beschriebenen Verfahren erfolgen.

Die Umsetzung wird bei Temperaturen von 10 bis 120 °C, d.h. bei Raumtemperatur oder bei höheren Temperaturen, zweckmäßig bei Temperaturen von 50 bis 120 °C, unter Atmosphärendruck oder in einem geschlossenen Gefäß unter erhöhtem Druck durchgeführt.

Die Ausgangsverbindungen der Formel II und III können ohne Verdünnungs- oder Lösungsmittel umgesetzt werden. Zweckmäßigerweise werden die Umsetzungen jedoch durchgeführt in Gegenwart eines inerten Verdünnungs- oder Lösungsmittels, beispielsweise eines niederen Alkohols mit 1 bis 4 C-Atomen,

wie Methanol, Äthanol oder Propanol, vorzugsweise Isopropanol oder Äthanol, eines niederen gesättigten Dialkyläthers, Dialkylglykoläthers oder cyclischen Äthers, wie Diäthyläther, 1,2-Dimethoxyäthan, Tetrahydrofuran oder Dioxan, eines Benzolkohlenwasserstoffs, wie Benzol selbst oder eines Alkylbenzols, insbesondere Toluol oder Xylol, oder eines aliphatischen Kohlenwasserstoffs, wie Hexan, Heptan oder Octan, Dimethylsulfoxid oder in Gegenwart von Wasser oder Mischungen der genannten Lösungsmittel.

Auch ist das in überschüssiger Menge verwendete Amin der allgemeinen Formel III gegebenenfalls als Verdünnungs- oder Lösungsmittel geeignet.

Die vollständige Umsetzung hängt von der Reaktionstemperatur ab und ist im allgemeinen innerhalb von 2 bis 15 Stunden beendet. Das Reaktionsprodukt kann in an sich üblicher Weise gewonnen werden, z.B. durch Filtration oder Abdestillieren des Verdünnungsmittels aus dem Reaktionsgemisch. Eine Reinigung der erhaltenen Verbindung erfolgt in üblicher Weise, beispielsweise durch Umkristallisation aus einem Lösungsmittel, Überführen in ein Säureadditionssalz oder durch Säulenchromatographie.

Der Phenoläther der allgemeinen Formel II kann durch Alkylie rung eines 2-Hydroxy-β-phenylpropiophenons der allgemeinen Formel IV

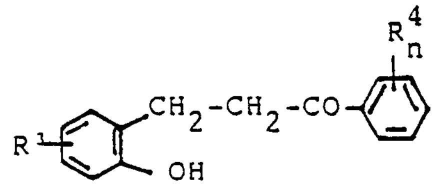

(IV)

mit einem Epihalogenhydrin erhalten werden. Als Epihalogenhydrine kommen Epichlorhydrin, Epibromhydrin und Epijodhydrin in Betracht.

Die Umsetzung der Verbindungen IV zur Herstellung der Ausgangsverbindungen der allgemeinen Formel II wird zweckmäßig bei Temperaturen von 0 bis 120 °C und unter Normaldruck oder in einem geschlossenen Gefäß unter erhöhtem Druck durchgeführt. Als Lösungs- oder Verdünnungsmittel werden zweckmäßig ein niederes aliphatisches Keton, wie Aceton, Methyläthylketon oder Methylisobutylketon, ein niederer Alkohol mit 1 bis 4 C-Atomen, wie Methanol, Äthanol, Propanol oder Butanol, ein niederer aliphatischer oder cyclischer Äther, wie Diäthyläther, Tetrahydrofuran oder Dioxan, ein Dialkylformamid, wie Dimethylformamid oder Diäthylformamid, oder Dimethylsulfoxid oder Hexamethylphosphorsäuretriamid oder überschüssiges Alkylierungsmittel verwendet.

Bevorzugt werden die Umsetzungen in Gegenwart einer Base als säurebindendes Mittel durchgeführt. Geeignete Basen sind Alkalimetallcarbonate, -hydrogencarbonate, -hydroxide, -hydride oder -alkoholate, insbesondere des Natriums und Kaliums, basische Oxide, wie Aluminiumoxid oder Calciumoxid, organische tertiäre Basen, wie Pyridin, niedere Trialkylamine, wie Trimethyl- oder Triäthylamin, oder Piperidin. Dabei können die Basen im Verhältnis zum eingesetzten Alkylierungsmittel in katalytischer Menge oder in stöchiometrischer Menge bzw. in geringem Überschuß verwendet werden.

Bevorzugt wird das 2-Hydroxy-β-phenylpropionphenon mit Epichlorhydrin oder Epibromhydrin in einem polaren, aprotischen Lösungsmittel, insbesondere Dimethylsulfoxid, in Gegenwart von mindestens einem Moläquivalent Base, insbesondere Natriumhydrid, bezogen auf das Alkylierungsmittel, bei Temperaturen von 0 bis 50 °C umgesetzt.

Die Ausgangsverbindung der allgemeinen Formel IV, d.h. das 2-Hydroxy-β-phenylpropiophenon, und seine Herstellung ist bekannt.

Gegebenenfalls wird die erhaltene erfindungsgemäße Verbindung der allgemeinen Formel I in ein Säureadditionssalz, vorzugsweise in ein Salz einer physiologisch verträglichen Säure überführt. Übliche physiologisch verträgliche anorganische und organische Säuren sind beispielsweise Salzsäure, Bromwasserstoffsäure, Phosphorsäure, Schwefelsäure, Oxalsäure, Maleinsäure, Fumarsäure, Milchsäure, Weinsäure, Äpfelsäure, Citronensäure, Salicylsäure, Adipinsäure und Benzoesäure. Weitere verwendbare Säuren sind beschrieben z.B. in Fortschritte der Arzneimittelforschung, Bd. 10, Seiten 224-225, Birkhäuser Verlag, Basel und Stuttgart, 1966, und Journal of Pharmaceutical Sciences, Bd. 66, Seiten 1-5 (1977). Bevorzugt ist Salzsäure.

Die Säureadditionssalze werden in der Regel in an sich bekannter Weise durch Mischen der freien Base oder deren Lösungen mit der entsprechenden Säure oder deren Lösungen in einem organischen Lösungsmittel, beispielsweise einem niederen Alkohol, wie Methanol, Äthanol, n-Propanol oder Isopropanol, oder einem niederen Keton, wie Aceton, Methyläthylketon oder Methyl-isobutylketon, oder einem Äther, wie Diäthyläther, Tetrahydrofuran oder Dioxan, erhalten. Zur besseren Kristallabscheidung können auch Mischungen der genannten Lösungsmittel verwendet werden. Darüber hinaus können pharmazeutisch vertretbare wäßrige Lösungen von Säure additionsverbindungen der Verbindung der Formel I in einer wäßrigen Säurelösung hergestellt werden.

Die Säureadditionssalze der Verbindung der Formel I können in an sich bekannter Weise, z.B. mit Alkalien oder Ionenaustauschern, in die freie Base übergeführt werden. Von der freien Base lassen sich durch Umsetzung mit anorganischen oder organischen Säuren, insbesondere solchen, die zur Bildung von therapeutisch verwendbaren Salzen geeignet sind, weitere Salze gewinnen. Diese oder auch andere Salze der neuen Verbindung, wie z.B. das Pikrat, können auch zur Reinigung der freien Base dienen, indem man die freie Base in ein Salz überführt, dieses abtrennt und aus dem Salz wiederum die Base freisetzt.

Gegenstand der vorliegenden Erfindung sind auch Arzneimittel zur oralen, rektalen, intravenösen oder intramuskulären Applikation, die neben üblichen Träger- und Verdünnungsmitteln eine Verbindung der Formel I oder deren Säureadditionssalz als Wirkstoff enthalten, sowie die Verwendung der neuen Verbindung und ihrer physiologisch verträglichen Salze bei der Behandlung von Herzrhythmusstörungen.

Die Arzneimittel der Erfindung werden mit den üblichen festen oder flüssigen Trägerstoffen oder Verdünnungsmitteln und den üblicherweise verwendeten pharmazeutisch-technischen Hilfsstoffen entsprechend der gewünschten Applikationsart mit einer geeigneten Dosierung in bekannter Weise hergestellt. Die bevorzugten Zubereitungen bestehen in einer Darreichungsform, die zur oralen Applikation geeignet ist. Solche Darreichungsformen sind beispielsweise Tabletten, Filmtabletten, Dragees, Kapseln, Pillen, Pulver, Lösungen oder Suspensionen oder Depotformen.

Selbstverständlich kommen auch parenterale Zubereitungen, wie Injektionslösungen, in Betracht. Weiterhin seien als Zubereitungen beispielsweise auch Suppositorien genannt.

Entsprechende Tabletten können beispielsweise durch Mischen des Wirkstoffs mit bekannten Hilfsstoffen, beispielsweise inerten Verdünnungsmitteln wie Dextrose, Zucker, Sorbit, Mannit, Polyvinylpyrrolidon, Sprengmittel wie Maisstärke oder Alginsäure, Bindemitteln wie Stärke oder Gelatine, Gleitmitteln wie Magnesiumstearat oder Talk und/oder Mitteln zur Erzielung eines Depoteffektes wie Carboxypolymethylen, Carboxymethylcellulose, Celluloseacetatphthalat oder Polyvinylacetat, erhalten werden. Die Tabletten können auch aus mehreren Schichten bestehen.

Entsprechend können Dragees durch Überziehen von analog den Tabletten hergestellten Kernen mit üblicherweise in Drageeüberzügen verwendeten Mitteln, beispielsweise Polyvinylpyrrolidon oder Schellack, Gummiarabicum, Talk, Titandioxid oder Zucker, hergestellt werden. Dabei kann auch die Drageehülle aus mehreren Schichten bestehen, wobei die oben bei den Tabletten erwähnten Hilfsstoffe verwendet werden können.

Lösungen oder Suspensionen mit dem erfindungsgemäßen Wirkstoff können zusätzlich geschmacksverbessernde Mittel wie Saccharin, Cyclamat oder Zucher sowie z.B. Aromastoffe wie Vanillin oder Orangenextrakt, enthalten. Sie können außerdem Suspendierhilfsstoffe wie Natriumcarboxymethylcellulose oder Konservierungsstoffe wie p-Hydroxybenzoate, enthalten. Wirkstoffe enthaltende Kapseln können beispielsweise hergestellt werden, indem man den Wirkstoff mit einem inerten Träger, wie Milchzucker oder Sorbit, mischt und in Gelatinekapseln einkapselt.

Geeignete Suppositorien lassen sich beispielsweise durch Vermischen mit dafür vorgesehenen Trägermitteln wie Neutralfetten oder Polyäthylenglykol bzw. deren Derivaten, herstellen.

Die Einzeldosierung liegt beim Menschen
für die orale Anwendung zwischen 0,5 - 5 mg/kg
für die i.v. Anwendung zwischen 0,05 - 2 mg/kg
für die i.m. Anwendung zwischen 0,1 - 3 mg/kg
für die rektale Anwendung zwischen 0,5 - 10 mg/kg.

Die erfindungsgemäßen Verbindungen und ihre physiologisch verträglichen Säureadditionssalze sind wegen ihrer antiarrhythmischen, β-sympatholytischen und Ca-antagonistischen Eigenschaften, vor allem zur Pharmakotherapie von Herzrhythmusstörungen zur Behandlung der koronaren Herzkrankheit und zur Prophylaxe des plötzlichen Herztodes geeignet. Die antiarrhythmische Wirksamkeit der erfindungsgemäßen Verbindungen wurde sowohl anhand von elektrophysiologischen Studien an Purkinje-Fasern aus Hundeherzen sowie mit Hilfe von Ouabain-induzierten ventrikulären Tachykardien bei Hunden bestimmt.

Die Wirkung auf die Kontraktionskraft des Herzens wurde am Meerschweinchenpapillarmuskel untersucht. Hämodynamische Studien wurden bei gesunden, wie auch akut infarzierten Hunden durchgeführt. In der folgenden Tabelle wird als wichtiges Kriterium der Sicherheitsfaktor (S.F.) dargestellt, der sich aus dem Verhältnis der antiarrhythmischen Wirksamkeit (Leitungsverzögerung C20_Vmax) zur negativen Inotropie (C20_CF) und der spezifisch stärkeren Wirksamkeit bei höheren Frequenzen (Rate-Faktor von Vmax) ergibt. Vergleichend sei hierbei erwähnt, daß die zur Zeit führenden Antiarrhythmika Propafenon und Flecainid ein S.F. von 1,5 bzw. 1,7 haben.

Als zusätzliches Kriterium wurde der Vorzeitigkeitsindex (Prematurity-Faktor) bestimmt, der die erwünschte Wirksamkeit der erfindungsgemäßen Verbindungen bei frühzeitig einfallenden Extrasystolen charakterisiert.

## Tabelle I

| Nr des Beispiels | C20_CF (µM) | C20_Vmax (µM) | Rate-Faktor von Vmax | Prematurity Faktor Vmax | S.F. |
|---|---|---|---|---|---|
| 1 | 15,0 | 12,5 | 5,00 | 0,50 | 6,0 |
| 2 | 8,0 | 3,6 | 1,27 | 1,15 | 2,8 |
| 4 | 22,0 | 4,8 | 1,13 | 1,01 | 5,2 |
| 5 | 12,0 | 4,4 | 1,39 | 0,98 | 3,8 |
| 6 | 20,0 | 11,3 | 1,32 | 0,70 | 2,3 |
| 7 | 18,0 | 5,7 | 1,39 | 0,98 | 4,4 |
| 8 | 13,0 | 8,3 | 1,34 | 0,97 | 2,1 |
| 9 | 25,0 | 10,2 | 1,30 | 0,97 | 3,2 |
| 12 | 18,0 | 5,8 | 1,14 | 0,90 | 3,5 |
| 16 | 9,0 | 11,9 | 5,00 | 0,50 | 3,8 |
| 24 | 10,0 | 1,6 | 1,15 | 1,01 | 7,2 |
| 26 | 3,0 | 1,7 | 1,16 | 0,93 | 2,1 |
| 27 | 4,0 | 1,6 | 0,99 | 1,24 | 2,5 |
| 38 | 7,0 | 1,7 | 1,30 | 0,97 | 5,4 |
| 44 | 6,0 | 2,6 | 1,11 | 0,99 | 2,6 |
| 46 | 2,0 | 2,2 | 2,50 | 0,92 | 2,3 |
| 47 | 2,0 | 0,3 | 1,08 | 0,99 | 7,2 |
| 48 | 3,0 | 1,0 | 1,18 | 0,91 | 3,5 |

-----

C20_CF: Konzentration, die die Kontraktion des Papillarmuskels um 20 % verringert

C20_Vmax: Konzentration, die Vmax bei einer Zykluslänge von 1000 mSek. um 20 % verringert

Rate-Faktor von Vmax: (% des Kontrollwertes Vmax bei einer Zykluslänge von 2000) / (% des Kontrollwertes Vmax bei einer Zykluslänge von 500) bei C20_Vmax

Prematurity Faktor Vmax: (% des Kontrollwertes Vmax bei vorzeitigen Extrasystolen) / (% des Kontrollwertes Vmax bei einem Normalschlag) bei Vmax

S.F.: Sicherheitsfaktor, errechnet als Rate-Faktor von Vmax * (C20_CF / C20_Vmax)

-----

Die Beispiele erläutern die Erfindung.

A) Herstellung der Ausgangsverbindungen

Beispiel I

1-(3,4-Dimethoxyphenyl)-3-[2'-(1,2-epoxy-3-propoxy)-phenyl]-1-propanon

275,4 g (0.96 Mol) 3,4-Dimethoxy-β-2'-hydroxyphenyl-propiophenon werden mit 600 ml Epichlorhydrin, 300 ml 2-Propanol und 38,5 g (0,97 Mol) Natriumhydroxid 5 h unter Rückfluß erhitzt und gerührt. Das entstandene Salz wird abfiltriert und die Lösung unter vermindertem Druck zur Trockene eingeengt. Der ölige Rückstand wird ohne Reinigung in die nächste Stufe eingesetzt. Ausbeute: 334,2 g ( 100 %) der Titelverbindung.

Analog wurden folgende Verbindungen hergestellt:

1-(3,4,5-Trimethoxyphenyl)-3-[2'-(1,2-epoxy-3-propoxy)-phenyl]-1-propanon
1-(3,4,5-Trimethylphenyl)-3-[2'-(1,2-epoxy-3-propoxy)-phenyl]-1-propanon
1-(2,4,6-Trimethylphenyl)-3-[2'-(1,2-epoxy-3-propoxy)-phenyl]-1-propanon
1-Phenyl-3-[2'-(1,2-epoxy-3-propoxy)-4'-methoxyphenyl]-1-propanon
1-(3-Methoxyphenyl)-3-[2'-(1,2-epoxy-3-propoxy)-phenyl]-1-propanon
1-(4-Methoxyphenyl)-3-[2'-(1,2-epoxy-3-propoxy)-phenyl]-1-propanon
1-(2-Methoxyphenyl)-3-[2'-(1,2-epoxy-3-propoxy)-phenyl]-1-propanon
1-(4-Methylphenyl)-3-[2'-(1,2-epoxy-3-propoxy)-phenyl]-1-propanon
1-Phenyl-3-[2'-(1,2-epoxy-3-propoxy)-phenyl]-1-propanon

B) Herstellung der erfindungsgemäßen Verbindungen

Beispiel 1

1-(3,4-Dimethoxyphenyl)-3-[2'-(2-hydroxy-3-n-propylamino-propoxy)-phenyl]-1-propanon-hydrochlorid

11,3 g (0,033 Mol) 1-(3,4-Dimethoxyphenyl)-3-[2'-(1,2-epoxy-3-propoxy)-phenyl]-1-propanon und 5,9 g (0,099 Mol) n-Propylamin werden in 100 ml Methanol gelöst und 3 1/2 h unter Rückfluß erhitzt. Das Lösemittel und das überschüssige Amin werden unter vermindertem Druck entfernt. Der ölige Rückstand wird in 150 ml Isopropanol gelöst und mit konzentrierter Salzsäure versetzt. Nach dem Erhitzen und Kühlen der Lösung werden Kristalle erhalten, die abgesaugt, getrocknet und aus Isopropanol umkristallisiert werden. Ausbeute 8,2 g (56 %) der Titelverbindung vom Fp. 126 °C.

Analog wurden hergestellt (Beispiele 2 bis 49):

2. 1-(3,4-Dimethoxyphenyl)-3-[2'-(2-hydroxy-3-tert.-pentylamino-propoxy)-phenyl]-1-propanon-hydrochlorid, Fp.: 151-152 °C.

3. 1-(3,4-Dimethoxyphenyl)-3-[2'-(2-hydroxy-3-morpholino-propoxy)-phenyl]-1-propanon-hydrochlorid, Fp.: 150-151 °C.

4. 1-(3,4-Dimethoxyphenyl)-3-[2'-(2-hydroxy-3-isopropylamino-propoxy)-phenyl]-1-propanon-hydrochlorid, Fp.: 135-136 °C.

5. 1-(3,4-Dimethoxyphenyl)-3-[2'-(2-hydroxy-3-tert.-butylamino-propoxy)-phenyl]-1-propanon-hydrochlorid, Fp.: 164-165 °C.

6. 1-(3,4-Dimethoxyphenyl)-3-[2'-(2-hydroxy-3-piperidino-propoxy)-phenyl]-1-propanon-hydrochlorid, Fp.: 124-125 °C.

7. 1-(3,4-Dimethoxyphenyl)-3-[2'-(2-hydroxy-3-(2''-hydroxypropylamino)-propoxy)-phenyl]-1-propanon-hydrochlorid, Fp.: 109-111 °C.

8. 1-(3,4-Dimethoxyphenyl)-3-[2'-(2-hydroxy-3-(2''-hydroxyethylamino)-propoxy)-phenyl]-1-propanon-hydrochlorid, Fp.: 146-147,5 °C.

9. 1-(3,4-Dimethoxyphenyl)-3-[2'-(2-hydroxy-3-(1''-hydroxybut-2-ylamino)-propoxy)-phenyl]-1-propanon-oxalat, Fp.: 195-197 °C.

10. 1-(3,4,5-Trimethoxyphenyl)-3-[2'-(2-hydroxy-3-tert.-pentylamino-propoxy)-phenyl]-1-propanon-hydrochlorid, Fp.: 179-180 °C.

11. 1-(3,4,5-Trimethoxyphenyl)-3-[2'-(2-hydroxy-3-n-propylamino-propoxy)-phenyl]-1-propanon-hydrochlorid, Fp.: 140-142 °C.

12. 1-(3,4,5-Trimethoxyphenyl)-3-[2'-(2-hydroxy-3-isopropylamino-propoxy)-phenyl]-1-propanon-hydrochlorid, Fp.: 161-162 °C.

13. 1-(3,4,5-Trimethoxyphenyl)-3-[2'-(2-hydroxy-3-tert.-butylamino-propoxy)-phenyl]-1-propanon-hydrochlorid, Fp.: 178,5-179,5 °C.

14. 1-(3,4,5-Trimethoxyphenyl)-3-[2'-(2-hydroxy-3-piperidino-propoxy)-phenyl]-1-propanon-hydrochlorid, Fp.: 148,5-150 °C.

15. 1-(3,4,5-Trimethoxyphenyl)-3-[2'-(2-hydroxy-3-morpholino-propoxy)-phenyl]-1-propanon-hydrochlorid, Fp.: 166,5-167,5 °C.

16. 1-(3,4,5-Trimethoxyphenyl)-3-[2'-(2-hydroxy-3-cyclohexylamino-propoxy)-phenyl]-1-propanon-hydrochlorid, Fp.: 158,5-159,5 °C.

17. 1-(3,4,5-Trimethylphenyl)-3-[2'-(2-hydroxy-3-piperidino-propoxy)-phenyl]-1-propanon-hydrochlorid, Fp.: 133-134 °C.

18. 1-(2,4,6-Trimethylphenyl)-3-[2'-(2-hydroxy-3-n-propylamino-propoxy)-phenyl]-1-propanon-oxalat, Fp.: 184-185 °C.

19. 1-(2,4,6-Trimethylphenyl)-3-[2'-(2-hydroxy-3-tert.-butylamino-propoxy)-phenyl]-1-propanon-oxalat, Fp.: 148-150 °C.

20. 1-(2,4,6-Trimethylphenyl)-3-[2'-(2-hydroxy-3-tert.-pentylamino-propoxy)-phenyl]-1-propanon-hydrochlorid, Fp.: 118-120 °C.

21. 1-(2,4,6-Trimethylphenyl)-3-[2'-(2-hydroxy-3-isopropylamino-propoxy)-phenyl]-1-propanon-hydrochlorid, Fp.: 111-112 °C.

22. 1-(2,4,6-Trimethylphenyl)-3-[2'-(2-hydroxy-3-morpholino-propoxy)-phenyl]-1-propanon-hydrochlorid, Fp.: 132-133 °C.

23. 1-Phenyl-3-[2'-(2-hydroxy-3-tert.-pentylaminopropoxy)-4'-methoxyphenyl]-1-propanon-oxalat, Fp.: 118-120 °C.

24. 1-(3-Methoxyphenyl)-3-[2'-(2-hydroxy-3-isopropylamino-propoxy)-phenyl]-1-propanon-hydrochlorid, Fp.: 109,5 °C.

25. 1-(3-Methoxyphenyl)-3-[2'-(2-hydroxy-3-tert.-pentylamino-propoxy)-phenyl]-1-propanon-hydrochlorid, Fp.: 113,5 °C.

26. 1-(3-Methoxyphenyl)-3-[2'-(2-hydroxy-3-n-propylamino-propoxy)-phenyl]-1-propanon-hydrochlorid, Fp.: 105,5 °C.

27. 1-(3-Methoxyphenyl)-3-[2'-(2-hydroxy-3-tert.-butylamino-propoxy)-phenyl]-1-propanon-hydrochlorid, Fp.: 110-111 °C.

28. 1-(3-Methoxyphenyl)-3-[2'-(2-hydroxy-3-morpholino-propoxy)-phenyl]-1-propanon-hydrochlorid, Fp.: 124,5 °C.

29. 1-(4-Methoxyphenyl)-3-[2'-(2-hydroxy-3-tert.-pentylamino-propoxy)-phenyl]-1-propanon-hydrochlorid, Fp.: 114,5-115,5 °C.

30. 1-(4-Methoxyphenyl)-3-[2'-(2-hydroxy-3-piperidino-propoxy)-phenyl]-1-propanon-hydrochlorid, Fp.: 151 °C.

31. 1-(4-Methoxyphenyl)-3-[2'-(2-hydroxy-3-n-propylamino-propoxy)-phenyl]-1-propanon-hydrochlorid, Fp.: 115,5-116,5 °C.

32. 1-(4-Methoxyphenyl)-3-[2'-(2-hydroxy-3-tert.-butylamino-propoxy)-phenyl]-1-propanon-hydrochlorid, Fp.: 142-143 °C.

33. 1-(2-Methoxyphenyl)-3-[2'-(2-hydroxy-3-isopropylamino-propoxy)-phenyl]-1-propanon-hydrochlorid, Fp.: 147-148 °C.

34. 1-(2-Methoxyphenyl)-3-[2'-(2-hydroxy-3-tert.-pentylamino-propoxy)-phenyl]-1-propanon-hydrochlorid, Fp.: 146-149 °C.

35. 1-(2-Methoxyphenyl)-3-[2'-(2-hydroxy-3-piperidino-propoxy)-phenyl]-1-propanon-hydrochlorid, Fp.: 107.5-109,5 °C.

36. 1-(2-Methoxyphenyl)-3-[2'-(2-hydroxy-3-(4''-methyl-1''-piperazinyl-propoxy)-phenyl]-1-propanon-hydrochlorid, Fp.: 172-175 °C.

37. 1-(2-Methoxyphenyl)-3-[2'-(2-hydroxy-3-n-propylamino-propoxy)-phenyl]-1-propanon-hydrochlorid, Fp.: 125-128 °C.

38. 1-(2-Methoxyphenyl)-3-[2'-(2-hydroxy-3-tert.-butylamino-propoxy)-phenyl]-1-propanon-hydrochlorid, Fp.: 145-147 °C.

39. 1-(2-Methoxyphenyl)-3-[2'-(2-hydroxy-3-morpholino-propoxy)-phenyl]-1-propanon-hydrochlorid, Fp.: 94-96 °C.

40. 1-(4-Methylphenyl)-3-[2'-(2-hydroxy-3-isopropylamino-propoxy)-phenyl]-1-propanon-hydrochlorid, Fp.: 126,5-128,5 °C.

41. 1-(4-Methylphenyl)-3-[2'-(2-hydroxy-3-cyclohexylamino-propoxy)-phenyl]-1-propanon-hydrochlorid, Fp.: 154-156 °C.

42. 1-(4-Methylphenyl)-3-[2'-(2-hydroxy-3-tert.-pentylamino-propoxy)-phenyl]-1-propanon-hydrochlorid, Fp.: 120,5-121,5 °C.

43. 1-(4-Methylphenyl)-3-[2'-(2-hydroxy-3-n-propylamino-propoxy)-phenyl]-1-propanon-hydrochlorid, Fp.: 109-111 °C.

44. 1-(4-Methylphenyl)-3-[2'-(2-hydroxy-3-tert.-butylamino-propoxy)-phenyl]-1-propanon-hydrochlorid, Fp.: 132,5-133,5 °C.

45. 1-Phenyl-3-[2'-(2-hydroxy-3-isopropylamino-propoxy)-phenyl]-1-propanon-hydrochlorid, Fp.: 85-90 °C.

46. 1-Phenyl-3-[2'-(2-hydroxy-3-cyclohexylamino-propoxy)-phenyl]-1-propanon-hydrochlorid, Fp.: 137-140 °C.

47. 1-Phenyl-3-[2'-(2-hydroxy-3-piperidino-propoxy)-phenyl]-1-propanon-hydrochlorid, Fp.: 132-133 °C.

48. 1-Phenyl-3-[2'-(2-hydroxy-3-tert.-butylamino-propoxy)-phenyl]-1-propanon-hydrochlorid, Fp.: 108-109 °C.

49. 1-Phenyl-3-[2'-(2-hydroxy-3-(3''-methoxypropylamino)-propoxy)-phenyl]-1-propanon-hydrochlorid, Fp.: 114-116 °C.

Beispiel 50

Herstellungsvorschrift für Tabletten

a) Rezeptur

| | |
|---|---|
| Verbindung von Beispiel 1 | 75,00 g |
| Mikrokristalline Cellulose (Pulver, 50 µm) | 15,75 g |
| Poly-(1-vinyl-2-pyrrolidon) | 5,00 g |
| Hydroxypropylmethylcellulose 2910 | 3,75 g |
| Magnesiumstearat | 0,50 g |
| | 100,00 g |

b) Mischen und Granulieren

Die Verbindung von Beispiel 1 wird bei Bedarf gesiebt, dann werden alle Rohstoffe außer Magnesiumstearat in einem Mischer gemischt und ebenfalls im Mischer mit einer geeigneten Menge einer Granulierflüssigkeit (z.B. Wasser oder Isopropanol-Dichlormethan 1:1) befeuchtet. Die feuchte Mischung wird mit einem geeigneten Sieb gesiebt, im Trockenschrank getrocknet und nochmals gesiebt. Das trockene Granulat wird mit dem Magnesiumstearat im Mischer vermengt.

c) Andeckmischung

| | |
|---|---|
| Talkum | 70,0 % |
| Calciumsulfat-Hemihydrat | 26,7 % |
| Hochdisperses Siliciumdioxid | 3,3 % |
| | 100,0 % |

d) Dragiersuspension

| | |
|---|---|
| Saccharose | 47,8 % |
| Talkum | 9,6 % |
| Calciumsulfat-Hemihydrat | 4,8 % |
| Arabisches Gummi | 3,6 % |
| Stärkesirup | 3,2 % |
| Macrogol® 6000 | 2,9 % |
| Titan (IV)-oxid | 1,6 % |
| Natriumdodecylsulfat | 0,1 % |
| dest. Wasser | 26,4 % |
| | 100,0 % |

Überziehen der Drageekerne

Die Drageekerne werden zunächst im rotierenden Kessel mit der Dragierlösung angefeuchtet und dann mit soviel Andeckmischung abgepudert bis sie wieder frei rollen. Nach dem Trocknen der Kerne wird dieser Vorgang wiederholt. Dann werden die Kerne im Trockenschrank nachgetrocknet. Danach werden die Kerne mit der Dragiersuspension schichtweise überzogen, bis das gewünschte Endgewicht erreicht ist. Jeweils nach dem Auftrag einer Schicht müssen die Kerne getrocknet werden.

Pressen von Tabletten

Aus der nach a) hergestellten Mischung werden in einer Tablettenpresse Tabletten mit einem Gewicht zwischen 40 und 400 mg gepreßt. Die Preßkraft und der Tablettendurchmesser werden so gewählt, daß die Zerfallzeit im Testgerät nach Ph. Eur. unter 15 Minuten liegt und die Tabletten mechanisch genügend stabil sind.

Beispiel 51

Herstellungsvorschrift für Filmtabletten

A. Rezeptur

a) Tablette
(siehe Herstellungsvorschrift für Tabletten, Beispiel 50)
b) Filmüberzug
Gesamtauftragsmenge 5-20 % des Tablettengewichts davon:
Hydroxypropylmethylcellulose 2910 77 %
Macrogol® 6000 (Weichmacher) 23 %

B. Herstellung der Tabletten

(siehe Herstellungsvorschrift für Tabletten, Beispiel 50)

C. Herstellung der Filmtabletten

Die Bestandteile des Filmüberzugs werden in einem geeigneten Lösungsmittel (z.B. Wasser oder Äthanol/Wasser 70:30) gelöst. Die Tabletten werden in einer Filmcoating-Anlage mit der Lösung des Filmbildners und des Weichmachers besprüht und im Heißluftstrom getrocknet. Die Filmtabletten werden im Trockenschrank nachgetrocknet.

Beispiel 52

Herstellungsvorschrift für Dragees

A. Rezeptur

a) Drageekern
(siehe Herstellungsvorschrift für Tabletten)
b) Drageedecke
Gesamtauftragsmenge 25-100 % des Kerngewichts davon:

| | |
|---|---|
| Saccharose | 51,4 % |
| Talkum | 24,0 % |
| Calciumsulfat-Hemihydrat | 10,3 % |
| Stärkesirup | 5,0 % |
| Arabisches Gummi | 3,9 % |
| Macrogol® 6000 | 2,9 % |
| Titan(IV)-oxid | 1,6 % |
| Hochdisperses Siliciumdioxid | 0,8 % |
| Natriumdodecylsulfat | 0,1 % |
| | 100,0 % |

B. Herstellung der Drageekerne

(siehe Herstellungsvorschrift für Tabletten)

C. Herstellung der Dragees Zusammensetzung der verwendeten Dragierlösung, Andeckmischung und Dragiersuspension

a) Dragierlösung

| | |
|---|---|
| Saccharose | 47,6 % |
| Stärkesirup | 19,1 % |
| Arabisches Gummi | 3,8 % |
| dest. Wasser | 29,5 % |
| | 100,0 % |

Beispiel 53

Herstellungsvorschrift für Kapseln

A. Wirkstoffdosierung ab 75 mg

1. Rezeptur

| | |
|---|---|
| Verbindung von Beispiel 2 | 75,00 g |
| Mikrokristalline Cellulose Pulver, 50 µm | 16,25 g |
| Poly-(1-vinyl-2-pyrrolidon) | 5,00 g |
| Hydroxypropylmethylcellulose 2910 | 3,75 g |
| | 100,00 g |

2. Mischen und Granulieren gemäß Beispiel 50b)

3. Abfüllen des Granulats in Kapseln

Das Granulat wird mit Hilfe einer Kapselfüllmaschine in Hartgelatinekapseln der Größe 3, 2, 1 oder 0 abgefüllt. Die Füllmenge pro Kapsel richtet sich dabei nach der gewünschten Dosierung des Wirkstoffes.

B. Wirkstoffdosierung 30-75 mg

1. Rezeptur

| | |
|---|---|
| Verbindung von Beispiel 4 | 30,00 - 75,00 g |
| Mikrokristalline Cellulose Pulver, 50 µm | 61,25 - 16,25 g |
| Poly-(1-vinyl-2-pyrrolidon) | 5,00 g |
| Hydroxypropylmethylcellulose 2910 | 3,75 g |
| | 100,00 g |

Die Summe der Einwaagen von Wirkstoff und mikrokristalliner Cellulose soll immer 91,25 g betragen. Die Wirkstoffmenge beträgt das Tausendfache der Einzeldosierung.

2. Mischen und Granulieren gemäß Beispiel 50b)

3. Abfüllen des Granulats in Kapseln

Je 100 mg Granulat werden mit Hilfe einer Kapselfüllmaschine in Hartgelatinekapseln der Größe 3 oder 2 abgefüllt.

Beispiel 54

Herstellungsvorschrift für Ampullen

1. Rezeptur

| | |
|---|---|
| Verbindung von Beispiel 8 | 1,5 g |
| Wasser für Injektionszwecke ad | 100,0 ml |

2. Herstellung der Lösung

90 % des für die gewählte Ansatzgröße benötigten Wassers werden vorgelegt; der Wirkstoff wird unter Erwärmen darin gelöst. Die Lösung wird nach dem Abkühlen auf das Endvolumen aufgefüllt.

3. Abfüllen der Lösung

Die fertige Lösung wird in Glasampullen, deren Füllmenge sich nach der gewünschten Dosierung richtet, abgefüllt. Sodann werden die Glasampullen zugeschmolzen.

4. Sterilisation

Die Ampullen werden 20 Minuten bei 120°C im Dampf sterilisiert.

Beispiel 55

Herstellungsvorschrift für Suppositorien

a. Rezeptur

| | |
|---|---|
| Verbindung von Beispiel 22 | 30 - 200 mg |
| Hartfett (Schmelzpunkt 35-36,5°C) ad | 2000 mg |

b. Herstellung der wirkstoffhaltigen Schmelze

Die für eine bestimmte Anzahl Suppositorien benötigte Menge Hartfett wird bei 40°C im Wasserbad geschmolzen. Der Wirkstoff wird durch ein 0,8 mm Sieb gedrückt und in die Schmelze eingerührt, so daß eine Suspension entsteht.

c. Herstellung der Suppositorien

Die Schmelze wird auf 37-38 °C abkühlen gelassen und unter ständigem Rühren in Zäpfchenformen so abgefüllt, daß das Gewicht eines Suppositoriums 2000 mg beträgt. Die Zäpfchenform wird nach dem Erstarren der Schmelze verschlossen.

## Ansprüche

1. Aminopropanolderivate von 3-(2-Hydroxyphenyl)-1-propanonderivaten der allgemeinen Formel I

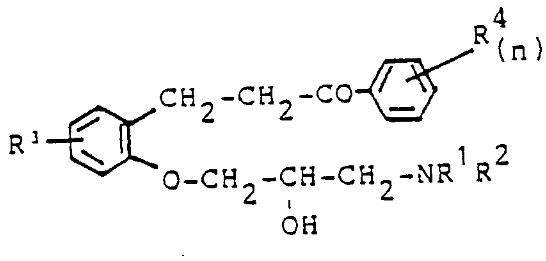

(I)

in der $R^1$ und $R^2$ gleich oder verschieden sind und Wasserstoffatome, Alkyl-, Cycloalkyl-, Alkenyl-, Alkinyl- oder Hydroxyalkylreste mit jeweils bis zu 6 C-Atomen, Alkoxyalkyl-, Alkylthioalkyl- oder Dialkylaminoalkylreste mit jeweils bis zu 9 C-Atomen oder Phenylalkyl- oder Phenoxyalkylreste mit bis zu 6 C-Atomen im Alkylteil und gegebenenfalls der Phenylrest durch einen Alkyl- oder Alkoxyrest mit jeweils bis zu 3 C-Atomen substituiert ist, bedeuten oder $R^1$ und $R^2$ zusammen mit dem sie verbindenden Stickstoffatom einen 5- bis 7-gliedrigen gesättigten heterocyclischen Ring bilden, der gegebenenfalls durch einen oder zwei Phenyl- und/oder Hydroxyreste substituiert sein und ein Sauerstoffatom als weiteres Heteroatom im Ring enthalten kann, wobei ein zusätzliches Stickstoffatom durch einen Alkylrest mit 1 bis 3 C-Atomen oder einen Phenylrest substituiert sein kann,
$R^3$ ein Wasserstoffatom, ein Alkylrest mit bis zu 3 C-Atomen, ein Fluor-, Chlor- oder Bromaton, eine Hydroxygruppe, eine Alkoxygruppe mit bis zu 6 C-Atomen ist,
$R^4$ ein Wasserstoffatom, ein Alkylrest mit bis zu 3 C-Atomen, ein Fluor-, Chlor- oder Bromatom, eine Hydroxygruppe, oder eine Alkoxygruppe mit bis zu 6 C-Atomen ist, und
n eine ganze Zahl mit einem Wert von 1 bis 5 ist, und ihre Säureadditionssalze.

2. Verbindungen nach Anspruch 1, dadurch gekennzeichnet, daß $NR^1R^2$ eine tert.-Pentylamino-, n-Propylamino-, 1,1-Dimethylpropylamino-, Morpholino-, Isopropylamino-, tert.-Butylamino-, Pyrrolidino-, Piperidino- oder Cyclohexylaminogruppe ist.

3. Verbindungen nach Anspruch 1 oder 2, dadurch gekennzeichnet, daß der Rest $R^3$ ein Wasserstoffatom, eine Methyl-, Methoxy- oder Hydroxylgruppe ist.

4. Verbindungen nach einem der Ansprüche 1 bis 3, dadurch gekennzeichnet, daß $R^4$ ein Wasserstoffatom, eine Methyl-, Methoxy- oder Hydroxylgruppe ist.

5. Verbindungen nach einem der Ansprüche 1 bis 4, dadurch gekennzeichnet, daß n den Wert 1, 2 oder 3 hat.

6. Verfahren zur Herstellung der Verbindungen nach Anspruch 1 und ihrer Säureadditionssalze, dadurch gekennzeichnet, daß man einen Phenoläther der allgemeinen Formel II

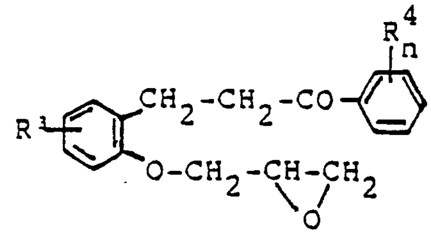

(II)

in der $R^3$, $R^4$ und n die in Anspruch 1 angegebene Bedeutung haben, mit einem Amin der allgemeinen Formel III

$HNR^1R^2$ (III)

in der $R^1$ und $R^2$ die in Anspruch 1 angegebene Bedeutung haben, umsetzt und gegebenenfalls die erhaltene Verbindung mit einer Säure in ein Säureadditionssalz überführt.

7. Arzneimittel, gekennzeichnet durch einen Gehalt an einer Verbindung gemäß Anspruch 1.

Europäisches Patentamt

# EUROPÄISCHER RECHERCHENBERICHT

Nummer der Anmeldung

EP 87 10 9015

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| Y | EP-A-0 075 207 (BASF)<br>* Ansprüche *<br>--- | 1,7 | C 07 C 93/06<br>C 07 D 295/08<br>A 61 K 31/13<br>A 61 K 31/395 |
| Y | US-A-4 540 697 (A. FRANKE)<br>* Spalten 1,2 *<br>--- | 1,7 | |
| Y | FR-A-2 362 115 (HEXACHIMIE)<br>* Ansprüche *<br>----- | 1,6,7 | |
| | | | **RECHERCHIERTE SACHGEBIETE (Int. Cl.4)** |
| | | | C 07 C 93/00<br>C 07 D 295/00<br>A 61 K 31/00 |

Der vorliegende Recherchenbericht wurde für alle Patentansprüche erstellt

| Recherchenort | Abschlußdatum der Recherche | Prüfer |
|---|---|---|
| DEN HAAG | 18-02-1988 | MOREAU J.M. |

**KATEGORIE DER GENANNTEN DOKUMENTE**

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPO FORM 1503 03.82 (P0403)